# EUROPÄISCHE PATENTANMELDUNG

(11) **EP 2 027 878 A1**
(43) Veröffentlichungstag der Anmeldung: **25.02.2009**
(21) Anmeldenummer: 07400020.9
(22) Anmeldetag: 18.08.2007
(51) Int. Cl.: A61M 5/28, A61F 17/00, A61K 31/167, A61K 31/245

(54) **Injektionsvorrichtung gefüllt mit Lokalanästhetikum zur Sofort-therapie von Insektenstichen für jedermann**

(71) Anmelder: Wührer, Klaus, 84359 Simbach/Inn (DE)
(72) Erfinder: Wührer, Klaus, 84359 Simbach/Inn (DE)
(74) Vertreter: Sporer, Christian

(57) **Zusammenfassung**

Die hier vorliegende Erfindung dient nun der sofortigen und wirksamen Bekämpfung und Therapie der Insektenstiche durch jedermann. Bestehend aus einer Einmalspritze (1), an der sich eine etwa ein bis maximal 2 mm lange Nadel befindet (3) ist vorgefertigt mit einem Lokalanästethikum, wie Prokain oder Lidokain in einer Lösung von 0,5 - 2 % mit maximal 0,5 ml gefüllt (2).

## Beschreibung

Die Erfindung dient der Soforttherapie für jedermann von Insektenstichen, bevorzugt Bienen-, Wespen-, Hummel- und Hornissenstichen durch intrakutan (in die Haut), im Bereich des Einstichs injiziertes Lokalanästhetikum (Lidokain oder Prokain) in nachfolgend spezifizierter Dosis zur Unterbindung der Histamin-Freisetzung im Körper und damit der Sofortbekämpfung der üblichen Symptome, wie Schmerz, Entzündung, Rötung, Schwellung, Juckreiz wie auch allergische Reaktionen.

Bisher werden Insektenstiche jeglicher Art bei Kindern, wie auch bei Erwachsenen, als Sofortmaßnahme durch Kühlung der Einstichstelle und Verwendung spezieller Salben und Gels sowie Einnahme von antihistaminischen Medikamenten behandelt. Diese Sofortbehandlungsmaßnahmen führen zumeist nur zu unzureichendem und langsamen Erfolg, erreichen jedoch nie die nahezu vollständige Unterbindung der bekannten Stichsymptome, wie akuter Schmerz, Entzündung, Rötung, Schwellung, Juckreiz usw.

Die im Schutzanspruch 1 angegebene Erfindung hilft diesem Problem ab.

Der Erfindung liegt insoweit die Überlegung zugrunde, eine Sofortinjektion eines Lokalanästhetikums in die Einstichstelle - durch jedermann - durchzuführen und damit vor allem die Histaminfreisetzung im Körper zu unterbinden und damit auch die vorgenannten Symptome umgehend zu bekämpfen und nahezu vollständig zu beheben bzw. deren Entstehung auszuschließen.

Die Erfindung ist insoweit so aufgebaut, dass standardisiert in ein zur Injektion verwendbares Behältnis (z. B. Einmalspritze) eine Menge von maximal 0,5 ml Lokalanästhetikum (herkömmliches in der Apotheke erhältliches Lokalanästhetikum wie Prokain oder Lidokain) in 0,5 - 2 %iger Lösung eingefüllt ist. An dem Injektionsbehältnis befindet sich eine 1 - maximal 2 mm lange Nadel, welche die Injektion in die Haut ausschließlich in die Einstichstelle des Insekts ermöglicht. Eine subkutane, das heißt unter die Haut gesetzte Injizierung wird dadurch ausgeschlossen. Diese Injektionsmöglichkeit bietet sich daher vom Aufbau und der Verwendbarkeit für jedermann an und muss nicht zwingend durch Arzt oder ähnliche Fachkräfte ausgeführt werden. Die Vorrichtung kann somit vorgefertigt in die Hausapotheke oder ein Notfallset vorrätig eingebracht werden und dann bei Bedarf umgehend seine Verwendung durch Jedermann finden.

Die Wirkung der Injektion entspricht dem Grundprinzip gängiger Schmerztherapien durch das genannte Lokalanästhetikum, erhöht jedoch die Wirkung durch die Direktzufuhr des Mittels. Damit wird das durch den Insektenstich verletzte Gewebe umgehend und direkt behandelt, so dass eine Reaktion des Gewebes durch Histaminausschüttung mit den bekannten und damit verbundenen Wirkungen, wie oben beschrieben, ausbleibt. Dies erfolgt erfahrungsgemäß bei einer Anwendung binnen 3 - 5 Minuten nach dem Insektenstich.

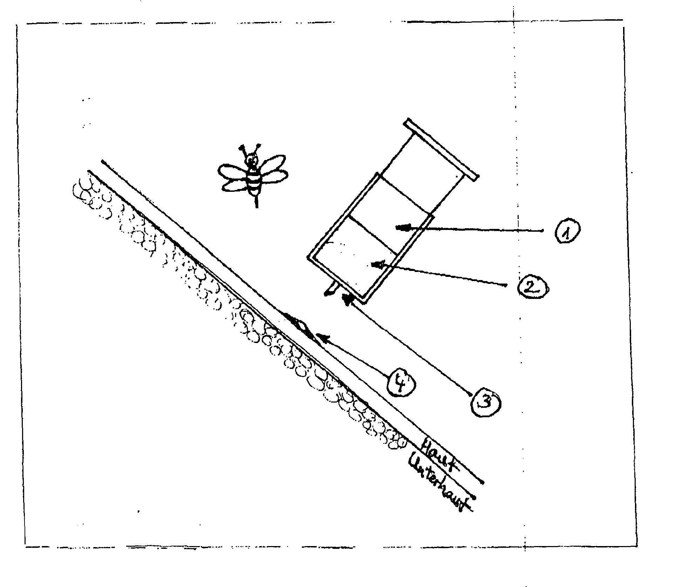

## Patentansprüche

1. Injektionsvorrichtung mit Lokalanästhetikum zur Soforttherapie von Insektenstichen,
**dadurch gekennzeichnet,**
**dass** ein Injektionsbehältnis (1) mit Lokalanästhetikum Lidokain oder Prokain (2) in einer Menge von maximal 0,5 ml in 0,5 - 2 %iger Lösung gefüllt ist und dieses Präparat durch die am Behältnis angebrachte 1 bis 2 mm lange Nadel (3) intrakutan, d. h. direkt in die Haut, zur akuten Behandlung von Insektenstichen injiziert werden kann.

2. Injektion nach Anspruch 1,
**dadurch gekennzeichnet,**
**dass** das Injektionsbehältnis (1) mit maximal 0,5 ml Lokalanästhetikum Prokain oder Lidokain (2) in 0,5 - 2 %iger Lösung gefüllt ist.

3. Injektion nach Anspruch 1,
**dadurch gekennzeichnet,**
**dass** an dem Injektionsbehältnis eine 1 bis 2 mm lange Injektionsnadel (3) befestigt ist und dadurch bei Injektion in die Haut ein Einbringen des Lokalanästhetikums direkt in den Stichbereich (4) gewährleistet und ein tieferes Eindringen der Nadel unter die Haut ausgeschlossen werden kann.

4. Injektion nach Anspruch 1,
**dadurch gekennzeichnet,**
**dass** die Injektion als Sofortmaßnahme durch jedermann erfolgen kann.
